# EUROPEAN PATENT SPECIFICATION

(11) **EP 3 423 836 B1**
(45) Date of publication and mention of the grant of the patent: **26.07.2023**
(21) Application number: 17710367.8
(22) Date of filing: 28.02.2017
(51) Int. Cl.: G01N 33/68

(54) **DETECTION OF MEMBRANE PROTEINS**
NACHWEIS VON MEMBRANPROTEINEN
DÉTECTION DE PROTÉINES MEMBRANAIRES

(30) Priority: 29.02.2016 GB 201603507
(43) Date of publication of application: 09.01.2019
(73) Proprietor: Oxford University Innovation Limited, Oxford, Oxfordshire OX2 0JB (GB)
(72) Inventor: HOPPER, Jonathan, Oxford Oxfordshire OX1 3TA (GB); ROBINSON, Carol, Oxford Oxfordshire OX1 3TA (GB); AMBROSE, Stephen, Oxford Oxfordshire OX1 3TA (GB)
(74) Representative: Drywood, Rosalind Aerona
(86) International application number: PCT/GB2017/050539
(87) International publication number: WO 2017/149294

(56) References cited:
- WO-A1-2012/172378
- WO-A1-2014/096821
- WO-A2-2005/094389
- MÁRIA KATONA ET AL: "A mass spectrometry based functional assay for the quantitative assessment of ABC transporter activity", RAPID COMMUNICATIONS IN MASS SPECTROMETRY., vol. 23, no. 21, 15 November 2009 (2009-11-15), pages 3372-3376, XP055368377, GB ISSN: 0951-4198, DOI: 10.1002/rcm.4259
- YANG DENG ET AL: "Fluidic and Air-Stable Supported Lipid Bilayer and Cell-Mimicking Microarrays", JOURNAL OF THE AMERICAN CHEMICAL SOCIETY, vol. 130, no. 19, 1 May 2008 (2008-05-01), pages 6267-6271, XP055368376, ISSN: 0002-7863, DOI: 10.1021/ja800049f
- FANG YE ET AL: "Membrane protein microarrays", JOURNAL OF THE AMERICAN CHEMICAL SOCIETY, THE SOCIETY, vol. 124, no. 11, 20 March 2002 (2002-03-20), pages 2394-2395, XP002512502, ISSN: 0002-7863, DOI: 10.1021/JA017346+
- STEPHEN AMBROSE ET AL: "Native Desorption Electrospray Ionization Liberates Soluble and Membrane Protein Complexes from Surfaces", ANGEWANDTE CHEMIE, INTERNATIONAL EDITION, vol. 56, no. 46, 18 September 2017 (2017-09-18), pages 14463-14468, XP055615723, DE ISSN: 1433-7851, DOI: 10.1002/anie.201704849
- MARHEINEKE K ET AL: "Lipid composition of Spodoptera frugiperda (Sf9) and Trichoplusia ni (Tn) insect cells used for baculovirus infection", FEBS LETTERS, ELSEVIER, AMSTERDAM, NL, vol. 441, no. 1, 11 December 1998 (1998-12-11), pages 49-52, XP004258869, ISSN: 0014-5793, DOI: 10.1016/S0014-5793(98)01523-3

## Description

### Field of the Invention

The present invention relates to the detection of membrane proteins. More particularly, the present invention relates to methods for the detection of membrane proteins using desorption electrospray ionisation mass spectrometry. The methods may be used to detect membrane proteins, including membrane proteins in the form of a complex in which the membrane protein is bound to a ligand such as a therapeutic agent.

### Background to the Invention

Membrane proteins are responsible for a wide range of biological functions. Some of the most prevalent human diseases, including some cancers, result from their dysfunction. Despite representing around a third of the human genome, membrane proteins represent targets for more than half of all current therapeutic agents. As a significant biological target in disease and cancer, their study by traditional structural biology approaches, such as X-ray crystallography and nuclear magnetic resonance, has been frustrated by limitations relating to their expression and solubility. Furthermore, X-ray analysis, in the majority of cases, has been limited by crystallographic resolution hindering the assignment of bound moieties.

More recently, mass spectrometry techniques have been developed which may be used to characterise membrane proteins and membrane protein complexes. In contrast to traditional structural biology methods, mass spectrometry is a rapid and sensitive technique that can be used to provide information on intact membrane proteins and their complexes (sometimes referred to as "native mass spectrometry").

Membrane proteins such as integral membrane proteins exhibit poor solubility. Accordingly, mass spectrometry has previously been performed on membrane proteins by using an electrospray solution in which the membrane protein has been solubilised in a detergent micelle. The electrospray solution may be prepared by buffer exchange of the purified membrane protein into a mass spectrometry-compatible buffer supplemented with the detergent. Once the electrospray solution has been prepared, it may be vaporised using a nanoelectrospray ionisation source under conditions such that the membrane protein is released from the micelle. The membrane protein is detected by a mass spectrometer. WO 2012/172378 and WO 2014/096821 disclose such processes.

Whilst representing a highly significant development in the field, the existing mass spectrometry methods are constrained by the use of the membrane protein in the electrospray solution. This means that the flexibility afforded by these methods is limited. For instance, in the case of screening for therapeutic agents that bind to a membrane protein, the nanoelectrospray ionisation source must be operated using a separate electrospray solution for each potential membrane protein-therapeutic agent complex.

Accordingly, there exists a need for mass spectrometry methods for detecting membrane proteins with greater flexibility. In particular, there exists a need for mass spectrometry methods that avoid the use of membrane proteins in the electrospray solution for a mass spectrometer.

Katona et al, Rapid Commun. Mass Spectrom. 2009, 23, 3372-3376 disclose a mass spectrometry based functional assay for the quantitative assessment of ABC transporter activity. Though the document discloses the use of desorption electrospray ionisation, this is not as part of a method in which a membrane protein is detected.

### Summary of the Invention

The present invention is based, at least in part, on a surprising discovery that a membrane protein may be desorbed from a surface on which a sample comprising a membrane protein and a solubilising agent has been deposited, ionised and detected using an desorption electrospray ionisation source coupled to a mass spectrometer. The invention enables the membrane protein to be detected in an intact 'native-like' state. In contrast to previous methods, the membrane protein is not introduced directly in the electrospray solution, but is rather in the form of a sample deposited on a surface to which electrospray is applied.

According to the present invention there is provided a method for detecting a membrane protein using a desorption electrospray ionisation source coupled to a mass spectrometer, wherein the method comprises:
desorbing a membrane protein from a surface on which a sample comprising the membrane protein and a solubilising agent is deposited by applying electrospray to the sample;
ionising the membrane protein; and
detecting the membrane protein using the mass spectrometer.

Also provided is a method for deriving information about a membrane protein using a desorption electrospray ionisation source coupled to a mass spectrometer, wherein the method comprises:
desorbing a membrane protein from a plurality of surfaces, on each of which a sample which comprises the membrane protein and a solubilising agent is deposited, by applying electrospray to the samples, wherein a different ligand and/or solubilising agent is present in the electrospray that is applied to each of the samples;
ionising the membrane proteins;
detecting the membrane proteins using the mass spectrometer; and
deriving information about the membrane protein based on the form in which the membrane protein is detected from each of the plurality of samples.

The present invention further provides a method for determining the dissociation constant of a complex which comprises a membrane protein bound to a ligand using a desorption electrospray ionisation source coupled to a mass spectrometer, wherein the method comprises:
desorbing membrane proteins from a plurality of surfaces, on each of which a sample which comprises the membrane protein, a ligand and a solubilising agent is deposited, each sample containing a different ratio of membrane protein to ligand, by applying electrospray to the samples;
ionising the membrane proteins;
detecting the membrane proteins using the mass spectrometer; and
calculating the dissociation constant of the complex based on the proportion of membrane proteins that are detected in the form of a complex in which the membrane protein is bound to the ligand from each of the samples.

### Brief Description of the Drawings

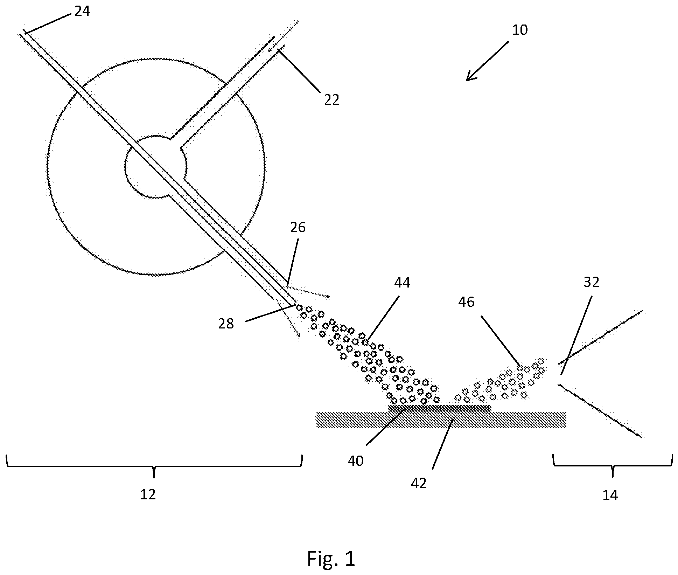

Figure 1 is a diagram of an apparatus 10 that may be used to carry out the method of the present invention. The apparatus comprises an electrospray ionisation source 20 coupled to a mass spectrometer 30. The electrospray ionisation source 20 comprises a nebulising gas source 22 and an electrospray solution inlet 24. Nebulising gas passes from the nebulising gas source 22 to a nebulising gas outlet 26. Electrospray solution passes from the electrospray solution inlet 24 to an electrospray outlet 28. The mass spectrometer 30 comprises an inlet 32. A surface 42 onto which a sample 40 comprising a membrane protein and a solubilising agent may be deposited is positioned between the electrospray ionisation source 20 and the mass spectrometer 30. The axis of the electrospray ionisation source 20 and the surface 42 define an angle θ, which is referred to as the spray angle.
   To carry out a method of the present invention employing the apparatus 10, a sample 40 is positioned on the surface 42 between the electrospray source 20 and the mass spectrometer 30. In use, nebulising gas and electrospray solution are delivered from their respective outlets 26, 28 to form an electrospray plume 44. The electrospray 44 is applied to the sample 40, and desorbed sample droplets 46 are generated. The desorbed sample droplets 46 pass through the inlet 32 to the mass spectrometer 30 for detection.

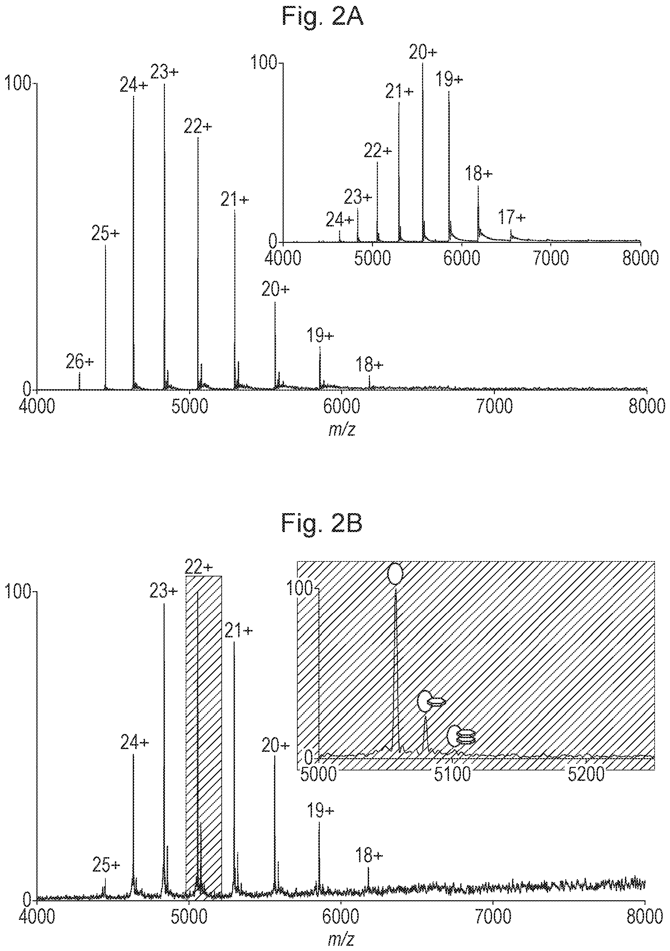

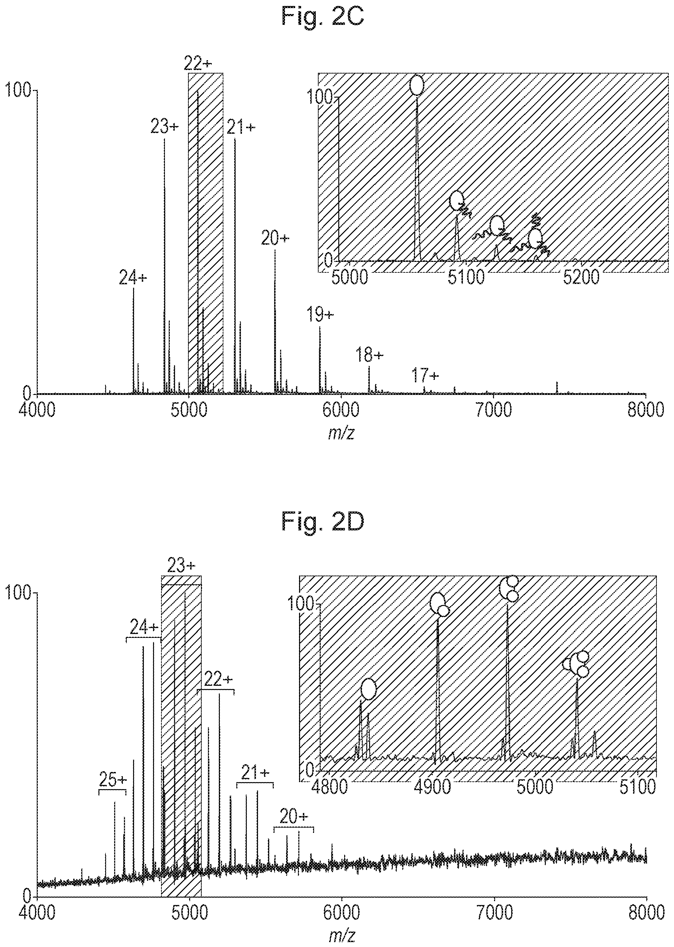

Figure 2 shows spectra of Outer Membrane Protein F (OmpF) desorbed from a solid surface and ionised using different electrospray ionisation solutions. Specifically, Figure 2 shows: A) spectra of OmpF desorbed and ionised using an electrospray solution containing octyl glucoside, a detergent (main), and ionised using a conventional nanospray process (inset); B) spectra (main and inset) of OmpF desorbed and ionised using an electrospray solution containing kanamycin sulfate, an antibiotic; C) spectra (main and inset) of OmpF desorbed and ionised using an electrospray solution containing 1-palmitoyl-2-oleoyl-*sn*-glycero-3-phospho-(1'-*rac*-glycerol) sodium salt, a lipid; and D) spectra (main and inset) of OmpF desorbed and ionised using an electrospray solution containing OBS, a synthetic peptide mimicking a colicin E9 binding epitope.

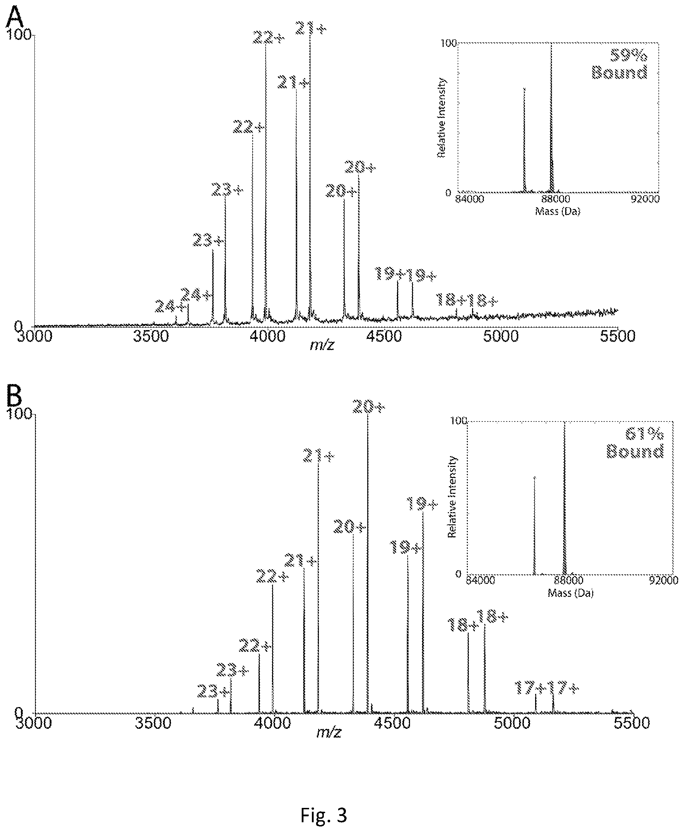

Figure 3 shows mass spectra of ferric hydroxamate receptor (FvpA) in complex with pyoverdine. Specifically, Figure 3 shows: A) spectra of FvpA desorbed from a solid surface and ionised using an electrospray solution; and B) spectra of FvpA obtained by vaporising and ionising a solution using conventional nanospray (insets show zero-charge deconvoluted spectra and calculated percentage bound).

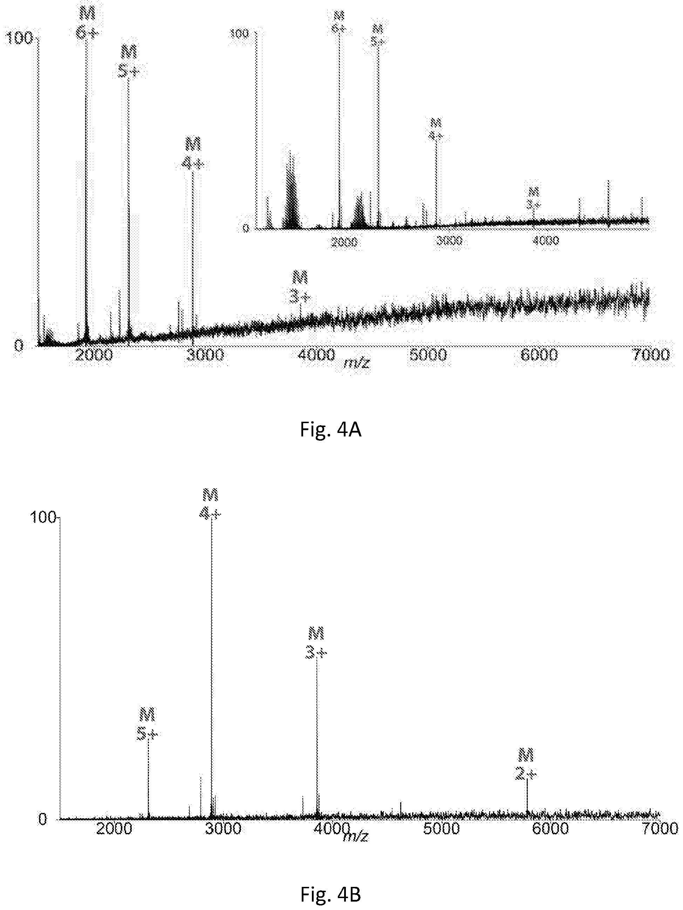

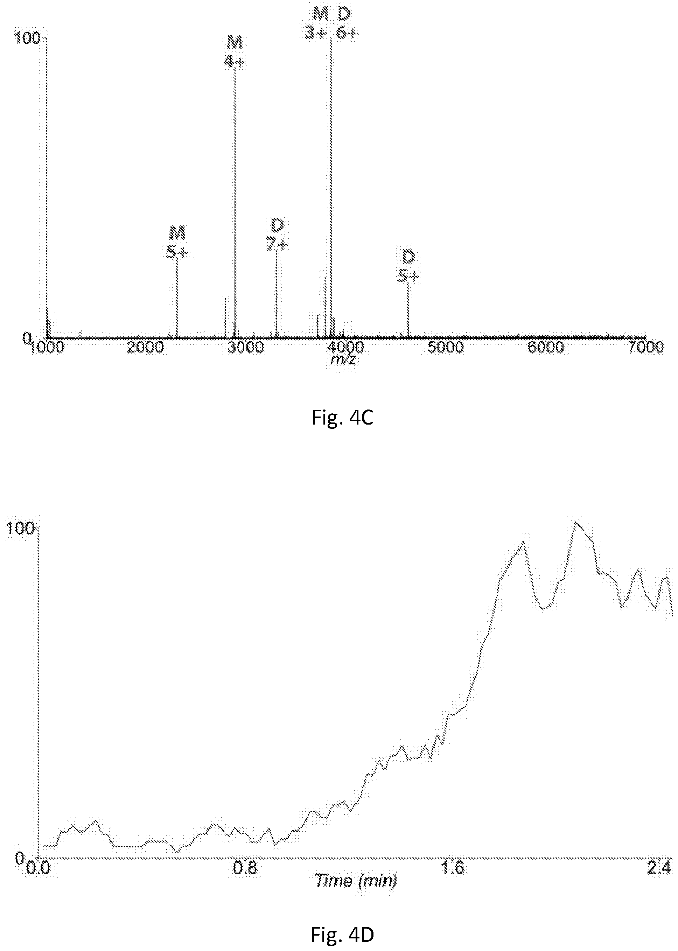

Figure 4 shows mass spectra of semiSWEET obtained using different detergents, as well as an extracted ion chromatogram of semiSWEET. Specifically, Figure 4 shows: A) a spectrum of semiSWEET desorbed from a solid surface and ionised using an electrospray solution containing *n*-dodecyl β-D-maltoside (main) and a spectrum of semiSWEET obtained by vaporising and ionising a solution using conventional nanospray (inset); B) a spectrum of semiSWEET desorbed from a solid surface and ionised using an electrospray solution containing lauryl dimethylamine-N-oxide; C) a spectrum of semiSWEET desorbed from a solid surface and ionised using an electrospray solution containing tetraethylene glycol monooctyl ether; and D) an extracted ion chromatogram of a 3303 m/z dimer peak from the semiSWEET desorbed using the electrospray solution containing tetraethylene glycol monooctyl ether.

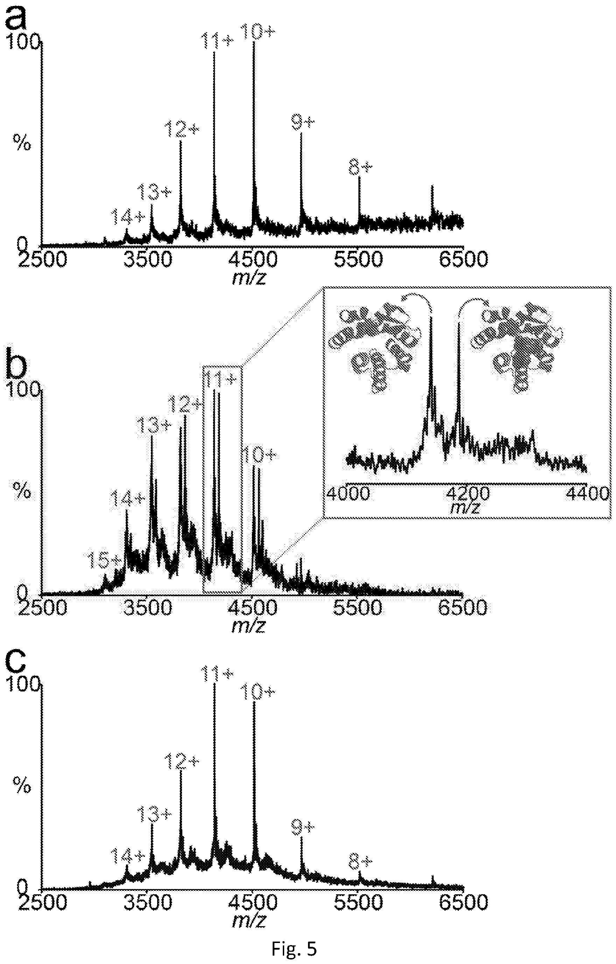

Figure 5a shows desorption electrospray ionisation (DESI) spectra of deposited GPCR P2Y, desorbed in a mixed micelle. Figure 5b shows competitive binding of MRS2500 to the protein GPCR P2Y₁ in a mixture of antagonists. Figure 5c shows a lack of binding observed when MRS2500 is removed from the mixture.

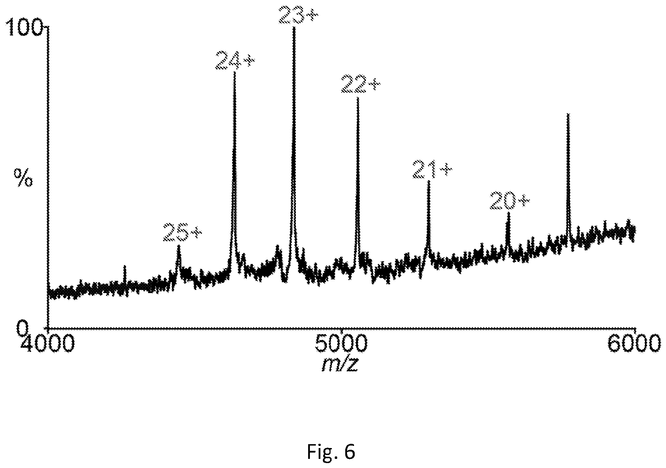

Figure 6 shows DESI spectra of deposited OmpF vesicles desorbed with 200 mM ammonium acetate and 1% OG solution.

### Description of Various Embodiments

The present invention provides a method for detecting a membrane protein using an electrospray ionisation source coupled to a mass spectrometer. The method involves desorbing a membrane protein from a surface on which a sample comprising the membrane protein and a solubilising agent is deposited by applying electrospray to the sample. The membrane protein is ionised, and subsequently detected using the mass spectrometer.

Membrane proteins can be grouped into integral membrane proteins and peripheral membrane proteins. Integral membrane proteins may have one or more segments embedded within a membrane and may be bound to the lipid bilayer. Peripheral membrane proteins may be temporarily associated with the lipid bilayer and/or integral membrane proteins. In an embodiment, the membrane protein that is used in the present invention is an integral membrane protein.

Membrane proteins may be composed of one (mono) or more (multi) associated polypeptide chains. Thus, the membrane protein may be a monomeric or a multimeric membrane protein, for example an oligomeric membrane protein. Oligomeric membrane proteins include both homooligomeric (identical polypeptide chains) and heterooligomeric (different polypeptide chains) proteins.

In an embodiment, the membrane protein is an integral membrane protein selected from G protein-coupled receptors (GPCRs), membrane transporters, membrane channels, ATP-binding cassette transporters (ABC-transporters) and proton driven transporters. In a particular embodiment, the membrane protein is selected from OmpF, FvpA, semiSWEET, EmrE, LmrP, MscL, BtuCD, BtuC₂D₂, LmrCD, MacB, MexB, P-gp, MsbA, peptide transporters, NorM and KirBac3.1.

In an embodiment, the membrane protein has a molecular weight of from about 10³ Daltons to about 10¹² Daltons, *e.g.* from about 10³ Daltons to about 10⁶ Daltons.

Methods for the purification and expression of membrane proteins are known in the art. By way of example, Barrera et al, Nat. Methods 2009, 6, 585-587 describe methods for the purification of MacB, LmrCD, and EmrE. Moreover, Drew et al, Nat. Protoc. 2008, 3, 784-798 describe a GFP fusion construct methodology in which yields in the overexpression and purification of membrane proteins are improved, while Aller et al, Science, 2009, 323, 1718-1722 describe P-glycoprotein expression and purification.

In addition to the membrane protein, a solubilising agent is present in the sample which is deposited on the solid surface. Solubilising agents are believed to stabilise deposition of the membrane protein on the surface and/or protect the membrane protein during desorption, ionisation and detection.

Suitable solubilising agents include lipids and detergents, with detergents preferably used. In embodiments, the sample may comprise both lipid and detergent solubilising agents.

Where the solubilising agent is a lipid, it may be present in the sample in the form of a lipid bilayer (*e.g.* a phospholipid bilayer). The use of a lipid bilayer enables the membrane protein to be studied in a more native-like environment. Suitable lipid bilayers include artificial bilayers. In an embodiment, the bilayer is selected from tethered membranes, vesicles, nanodiscs, bicelles, lipodisqs (also known as SMALPs) and lipidic cubic phases. In an embodiment, the membrane protein is embedded in the lipid bilayer in the sample. Preparation of bilayers having membrane proteins embedded therein is disclosed in Deng et al, J. Am. Chem. Soc. 2008, 130, 6267-6271. In an embodiment, when the electrospray is applied to the sample, some of the lipid bilayer may be desorbed with the membrane protein, and the membrane protein may be ionised and detected in the form of a complex in which the membrane protein is bound to one or more lipids.

Where the solubilising agent is a detergent, the detergent is preferably a non-ionic detergent, as high concentrations of non-ionic detergents can be tolerated more readily during the electrospray process. Suitable non-ionic detergents include *n*-dodecyl β-D-maltoside, glycosides, neopentyl glycols, polyoxyethylene glycols, facade EM, maltosides, glucosides (such as octyl glucoside and nonyl glucoside), and mixtures thereof. Preferably, the detergent comprises *n*-dodecyl β-D-maltoside.

Preferably, a ligand is present in the sample and/or the electrospray. In an embodiment, a method of the present invention may comprise ionising and detecting the membrane protein in the form of a complex in which the membrane protein is bound to the ligand. Thus, a method of the present invention may be used to detect binding between a membrane protein and a ligand. In particular, a method of the present invention may allow one or more structural characteristics (*e.g.* stoichiometry) of a membrane protein-ligand complex to be determined, and/or may also be used to detect conformational changes that take place upon binding of the ligand to the membrane protein.

In an embodiment, the ligand is present in the sample which comprises the membrane protein and the solubilising agent. Preferably, the sample comprises a complex of the ligand and the membrane protein. In another embodiment, the membrane-protein-ligand complex may be formed by adding the ligand to the sample via the electrospray.

A method of the present invention may therefore allow ligands to be screened. In contrast to indirect methods such as fluorescence or calorimetry, the present method may allow ligands to be screened directly. In particular, a method may be used to screen for the binding of activators and transporter substrates which are difficult to screen using conventional *in vivo* methodologies. Moreover, unlike X-ray crystallography, the present methods are not complicated by the inherent structural flexibility of membrane protein-ligand complexes and may allow the dynamical behaviour of membrane proteins and their interaction with ligands to be studied.

Ligands which may be used in the methods of the present invention include therapeutic agents, nucleotides and lipids. In a preferred embodiment, the ligand is a therapeutic agent.

The therapeutic agent may be an active compound which, when administered to an organism (human or non-human animal), induces a desired pharmacologic, immunogenic, and/or physiologic effect by local and/or systemic action. Examples of therapeutic agents include, without limitation, drugs, vaccines and biopharmaceutical agents. Thus, therapeutic agents may include small molecule drugs, therapeutic proteins (including antibodies), peptides and fragments thereof (whether naturally occurring, chemically synthesised or recombinantly produced), and nucleic acid molecules (including both double-and single-stranded molecules, gene constructs, expression vectors, antisense molecules and the like). Therapeutic agents may also include substrates, inhibitors, activators, neurotransmitters, agonists and antagonists. The therapeutic agent may be a synthetic or naturally occurring compound. The therapeutic agent may be a drug candidate or other agent suspected of having therapeutic application.

Particular examples of therapeutic agents include, but are not limited to, anti-cancer agents, anti-infective agents (*e.g.* antibiotics and antiviral agents), analgesic agents, anorexic agents, anti-inflammatory agents, antiepileptic agents, anaesthetic agents, hypnotic agents, sedatives, antipsychotic agents, neuroleptic agents, antidepressants, anxiolytics, antagonists, neuron blocking agents, anticholinergic and cholinomimetic agents, antimuscarinic and muscarinic agents, antiadrenergics agents, hormones, nutrients, antiarthritics agents, antiasthmatic agents, anticonvulsants, antihistamines, antinauseants agents, antineoplastic agents, antipruritics agents, antipyretic agents; antispasmodic agents, cardiovascular agents (*e.g.* calcium channel blockers, beta-blockers, beta-agonists, antiarrhythmic agents, antihypertensive agents, diuretics and vasodilators), central nervous system stimulants; decongestants, hormones, bone growth stimulants, bone resorption inhibitors, immunosuppressive agents, muscle relaxants, psychostimulants, sedatives and tranquilisers. It will be appreciated that this list of therapeutic agents is merely illustrative and should not be considered to be limiting. Many other therapeutic agents are known in the art and may be utilised in a method of the present invention. A detailed description of various therapeutic agents may be found in e.g. Remington's Pharmaceutical Sciences (21st edition, 2005, Mack Publishing Company). The therapeutic agent may exhibit optical isomerism and/or diastereoisomerism. Accordingly, the therapeutic agent may be in the form of a single enantiomer or diastereoisomer, or a mixture (*e.g.* a racemic mixture) thereof.

In an embodiment, the therapeutic agent has a molecular weight of less than 2000 Daltons, *e.g.* less than 1500 Daltons, *e.g.* less than 1000 Daltons, *e.g.* less than 500 Daltons. In an embodiment, the therapeutic agent is a non-polymeric organic compound having a molecular weight of less than 1000 Daltons, *e.g.* less than 800 Daltons, *e.g.* less than 500 Daltons.

In an embodiment, the therapeutic agent is an inhibitor or an activator, e.g. an activator or inhibitor of the membrane protein to which it is bound. In a particular embodiment, the therapeutic agent is a cyclic peptide inhibitor. Examples of cyclic peptide inhibitors include QZ59Se and HT-55. Examples of activators include HT-44 and HT-122.

In an embodiment, the therapeutic agent is an anti-cancer agent. In a particular embodiment, the therapeutic agent is selected from doxorubicin, digoxin, loperamide, berberine, irinotecan, vinblastine, paclitaxel, fexofenadine, cyclosporin, quinidine, tariquidar and verapamil. In an embodiment, the therapeutic agent is cyclosporin A.

Other suitable ligands for use in the present invention include nucleotides and lipids.

In an embodiment, more than one ligand may be present in the sample and/or the electrospray. For example, a therapeutic agent and a nucleotide or lipid may be present in the sample and/or the electrospray. Thus, a method of the present invention may be used to determine whether the presence of a ligand, *e.g.* a nucleotide or lipid, affects binding of another ligand, *e.g.* a therapeutic agent, to the membrane protein. The membrane protein may be ionised and detected in the form of a complex in which the membrane protein is bound to a therapeutic agent and one or more further ligands.

In an embodiment, more than one ligand, *e.g.* a plurality of different therapeutic agents, may be present in the electrospray. Thus, a method of the present invention may be used to screen ligands, such as therapeutic agents, for their affinity with the membrane protein. In an embodiment, at least four, *e.g.* at least six, *e.g.* at least eight ligands are used. In an embodiment, the plurality of ligands is a library of therapeutic agents. The membrane protein may be ionised and detected in the form of a complex in which the membrane protein is bound to at least one of the ligands.

In an embodiment, a molar excess of the ligand is used as compared to the membrane protein. In an embodiment, the molar ratio of the ligand to the membrane protein is at least 2:1, *e.g.* at least 5:1, *e.g.* at least 10:1. However, the ligand may be present in far higher molar ratios, particularly where it is added to the membrane protein via the electrospray. Lower ratios of ligand to membrane protein may also be used, *e.g.* less than 1:1, e.g. less than 1:2.

Binding of the ligand to the membrane protein may be via a non-covalent or a covalent interaction. In particular, binding of the ligand to the membrane protein may be via intermolecular forces such as ionic bonds, hydrogen bonds and van der Waals forces. Binding of the ligand to the membrane protein may be reversible or irreversible. In an embodiment, the ligand is bound to the membrane protein via a reversible bond.

The membrane protein is desorbed from a surface on which the sample is deposited. The sample may be deposited on any surface provided that it does not interfere with the species that may be detected by the mass spectrometer. Suitable materials for the surface include solid surfaces, such as glass or plastic. The sample will typically be deposited on a substantially flat surface. In an embodiment, the surface may be functionalised to capture samples of interest, immobilising them to the surface. In an embodiment, the surface comprises an electron microscopy grid.

The sample may be a liquid or a solid sample. In a preferred embodiment, the sample is a solid sample.

A liquid sample may be a solution in which the membrane protein and solubilising agent are dissolved, or it may be a liquid sample which comprises the membrane protein and solubilising agent in an undissolved state.

In some embodiments, the liquid sample is a solution which comprises a detergent micelle in which the membrane protein is contained. The solution will typically comprise a plurality of micelles. Where a ligand is present, the solution preferably comprises a detergent micelle in which the membrane protein is contained in the form of a complex with the ligand. The solution may be formed *e.g.* by incubating the membrane protein with the ligand in the presence of a detergent.

In order to minimise precipitation of the components in the solution, as well as dissociation of any membrane protein-ligand complexes that are present, the detergent should be present in the micellar solution at a concentration at least equal to, and preferably greater than, the critical micelle concentration (CMC) of the detergent. The critical micelle concentration of the detergent may be determined experimentally using methods known in the art, or it may be obtained from e.g. a textbook, product catalogue or website. For example, the Anatrace products catalogue provides CMC data. Typically, critical micelle concentration values are determined in water at 25 °C. Thus, in an embodiment, the solution is an aqueous solution and the detergent is present in the solution at a concentration at least equal to, and preferably greater than, the critical micelle concentration of the detergent in water at 25 °C.

In an embodiment, the detergent is present in the solution at a concentration of from about 100 µM to about 100 mM, e.g. from about 100 µM to about 200 µM. In an embodiment, the membrane protein is present in the solution at a concentration of from about 0.1 µM to about 500 µM, e.g. from about 5 µM to about 50 µM. Where the ligand is present in the solution, it may be present in the solution at a concentration of at least 2 µM, *e.g.* at least 5 µM.

The liquid sample may be deposited on a surface, e.g. using a syringe or pipette. In another embodiment, the liquid sample may be deposited on a surface by spraying the liquid sample onto the surface, *e.g.* by vaporising the liquid sample using a nebulising gas, *e.g.* from an electrospray ionisation source.

The liquid sample may have may have a volume of less than 1 ml, *e.g.* less than 0.5 ml, *e.g.* less than 0.1 ml.

It has surprisingly been found that methods of the present invention may be carried out using a solid sample which comprises a membrane protein and a solubilising agent. In particular, it is surprising that membrane proteins, which exhibit poor solubility, may be at least partially solubilised, desorbed and ionised from a solid sample, and subsequently detected, *e.g.* in a form in which the membrane protein is substantially intact.

The solid sample may contain liquid components in a total amount of less than 20 % by weight, *e.g.* less than 10 % by weight, *e.g.* less than 5 % by weight. Preferably the solid sample is substantially free from any liquid components.

The solid sample may be obtained by a method in which a liquid sample, such as a liquid sample as described above, is deposited on a surface, and the solvent is removed. In an embodiment, the method comprises preparing the solid sample by removing the solvent from a liquid sample which comprises a solubilising agent and a membrane protein, such as a liquid sample as described above. Without wishing to be bound by theory, it is believed that the presence of a solubilising agent helps to stabilise the membrane protein during removal of the solvent. Where a ligand is present in the solid sample, then the solid sample is preferably obtained from a liquid sample which also comprises the ligand. In an embodiment, the membrane protein is present in the solid sample in the form of a complex comprising the membrane protein bound to the ligand.

The dehydrated sample may be prepared by applying a droplet of the liquid sample to a surface, *e.g.* using the methods described above. The droplet may have a volume of less than 1 ml, *e.g.* less than 0.5 ml, *e.g.* less than 0.1 ml. The solvent may be removed from the droplet by evaporation under ambient conditions, or by slightly heating the droplet e.g. using warm air. Preferably, the solvent is removed be passing nebulising gas, e.g. from the electrospray ionisation source, over the sample.

The method of the present invention is carried out by applying electrospray from the electrospray ionisation source to the sample. The electrospray ionisation source may comprise a nebulising gas source and a solution inlet. The nebulising gas, with an applied voltage, transforms the solution into electrospray. The electrospray ionisation source is operated under conditions such that the membrane protein is desorbed from the surface on which the sample is deposited, ionised and transferred to the mass spectrometer for detection.

The electrospray may comprise one or more conventional electrospray components, such as a buffer. Ammonium acetate is particularly preferred in this regard. The concentration of ammonium acetate is preferably at least 10 mM. Preferably the pH of the buffer is in the range of from 5 to about 8. In an embodiment, solutions which lead to protein denaturation may be used, *e.g.* solutions containing organic solvents such as methanol, acetonitrile or acids *e.g.* acetic or formic acid.

In an embodiment, the electrospray comprises a solubilising agent, such as a detergent or a lipid. The solubilising agent may be selected from the solubilising agents described above as suitable for use as the solubilising agent in the sample. In embodiments, the electrospray may comprise both detergent and lipid solubilising agents.

The same or different solubilising agents may be used in the electrospray and the sample.

A detergent is preferably present in the electrospray, e.g. at a concentration at least equal to, and preferably greater than, the critical micelle concentration (CMC) of the detergent. The CMC may be determined using methods discussed above. This helps to maintain a good concentration of detergent in the sample throughout the desorption process. In an embodiment, the detergent is present in the electrospray at a concentration of from about 100 µM to about 100 mM, *e.g.* from about 100 µM to about 200 µM.

As mentioned above, the electrospray may also comprise one or more ligands. Where the ligand is present in the electrospray solution, it may be present at a concentration of at least 0.5 µM, *e.g.* at least 2 µM, *e.g.* at least 5 µM.

In preferred embodiments, a detergent is present as a solubilising agent in at least one of the sample and the electrospray. It is believed that, during application of the electrospray, detergent (in the sample or added via the electrospray) may form a micelle which encapsulates the membrane protein. Without wishing to be bound by theory, it is believed that the detergent, *e.g.* in the form of a micelle, may provide at least partial shielding during the electrospray ionisation process and, in the case of a dried sample, may help with at least partial solubilisation of the membrane protein by the electrospray. The electrospray ionisation source and mass spectrometer are operated under conditions such that the membrane protein is released from any micelles that have formed before detection.

Ionisation may occur during desorption of the membrane protein from the surface on which the sample is deposited. It is believed that ionisation of the membrane protein generally occurs during or after release of the membrane protein from any micelle that may be present. In some instances, however, portions of the membrane protein, e.g. hydrophilic/cytoplasmic domains, may become ionised prior to release of the membrane protein from any micelle.

In a preferred embodiment, the membrane protein is maintained in an intact, folded state throughout the desorption and ionisation process. This may allow the membrane protein to be detected by the mass spectrometer in a "native" state. In another embodiment, the membrane protein is detected in a partially folded or unfolded state.

The membrane protein is detected using a mass spectrometer. It will be appreciated that the membrane protein that is detected by the mass spectrometer is in an ionised state. A mass spectrometer will typically comprise a mass analyser and a detector. Thus, the step of detecting the membrane protein using the mass spectrometer may comprise resolving the membrane protein (in its ionised state) using the mass analyser and detecting the resolved membrane protein using the detector.

The mass spectrometer is preferably adapted to transmit and detect ions having mass-to-charge (*m*/*z*) ratios in the range of *e.g.* from about 100 *m*/*z* to about 32,000 *m*/*z.* Preferably, the mass spectrometer is operated under conditions suitable for maintaining and focusing large macromolecular ions. Preferably, the mass spectrometer is capable of providing a relatively high collisional activation energy to improve the removal of solubilising agents (including *e.g.* any micelles). This may improve the resolution of protein peaks, by preventing the mass spectrum from being dominated by peaks attributable to the solubilising agent. By way of illustration, and without limitation, the mass spectrometer may be an orbitrap mass spectrometer, such as a ThermoScientific^{™} Q-Exactive mass spectrometer. The resolution provided by such instruments is particularly suited to resolving peaks generated from a membrane protein, including a membrane protein in the form of a complex in which the membrane protein is bound to a ligand.

Together, the electrospray ionisation source and the mass spectrometer form a desorption electrospray ionisation (DESI) apparatus.

The electrospray ionisation source may be operated under one or more of the following conditions: (i) a spray voltage of from about 1 to about 10 kV, *e.g.* from about 2 to about 7 kV, *e.g.* from about 4 to about 5 kV; (ii) a solvent flow rate of from about 0.5 to about 5 µL/min, *e.g.* from about 1 to about 4 µL/min, *e.g.* from about 2 to about 3 µL/min; (iii) a nebulising gas pressure of from about 500 to about 1500 kPa, *e.g.* from about 650 to about 1200 kPa, *e.g.* from about 800 to about 1000 kPa; (iv) a spray angle of from about 30 to about 75 °, *e.g.* from about 45 to about 70 °, *e.g.* from about 55 to about 65 °; and (v) a spray to sample distance of from about 0.5 to about 8 mm, *e.g.* from about 1 to about 6 mm, *e.g.* from about 2 to about 4 mm.

The mass spectrometer may be operated under one or more of the following conditions: (i) a sample to capillary distance of from about 0.5 to about 5 mm, *e.g.* from about 0.75 to about 3 mm, *e.g.* from about 1 to about 2 mm; (ii) an extended trapping energy of from about 50 to about 300 eV, *e.g.* from about 75 to about 250 eV, *e.g.* from about 100 to 200 eV; (iii) a source fragmentation energy of from about 0 to about 200 eV, *e.g.* from about 0 to about 150 eV, *e.g.* from about 0 to about 100 eV; and (iv) a high vacuum pressure of from about 0.6 × 10⁻⁴ to about 6 × 10⁻⁴ mPa , *e.g.* from about 0.8 × 10⁻⁴ to about 4 × 10⁻⁴ mPa, *e.g.* from about 1 × 10⁻⁴ to about 2 × 10⁻⁴ mPa.

The membrane protein is then resolved and detected using the mass spectrometer and, if desired, further characterised. In this regard, the present methods are desirable in that the membrane protein, or a fragment thereof, in the form of a complex in which the membrane protein is bound to a ligand can be detected directly using the mass spectrometer, rather than inferred indirectly from mass spectra of the separate components (ligand and protein). Moreover, where the membrane protein-ligand complex comprises further components, *e.g.* a lipids or nucleotides, the binding of one or more of said components to the membrane protein may be detected simultaneously. For instance, the method may comprise detecting the membrane protein, or a fragment thereof, in a plurality of forms selected from the group consisting of: a complex in which the membrane protein, or a fragment thereof is bound to a therapeutic agent; a complex in which the membrane protein, or a fragment thereof is bound to one or more additional components (*e.g.* selected from lipids and nucleotides); and a complex in which the membrane protein, or a fragment thereof is bound to a therapeutic agent and one or more additional components (*e.g.* selected from lipids and nucleotides). Thus, the present methods may be used to detect concomitant binding of the membrane protein with a plurality of components, *e.g.* ligands or solubilising agents, which compete for binding sites.

As mentioned above, by applying electrospray to a sample containing a membrane protein which is deposited on a surface, flexibility is imparted onto mass spectrometry methods. In particular, the methods of detecting a membrane protein that are disclosed herein may readily be carried out on a plurality of samples, with each sample and/or the electrospray that is applied to each sample being different. In this way, mass spectrometry methods may be used for deriving information about a membrane protein, or for determining the dissociation constant of a complex which comprises a membrane protein bound to a ligand.

For example, a method for deriving information about a membrane protein using a desorption electrospray ionisation source coupled to a mass spectrometer may comprise: desorbing a membrane protein from a plurality of surfaces, on each of which is deposited a sample which comprises the membrane protein and a solubilising agent, by applying electrospray to the samples, wherein a different ligand and/or solubilising agent is present in the electrospray that is applied to each of the samples; ionising the membrane proteins; detecting the membrane proteins using the mass spectrometer; and deriving information about the membrane protein based on the form in which the membrane protein is detected from each of the plurality of samples.

The plurality of surfaces may together form one larger surface, *i.e.* the plurality of surfaces may be areas of a larger surface. It will be appreciated that the same membrane protein is present in each of the samples. In an embodiment, the plurality of surfaces includes at least two, *e.g.* at least four, *e.g.* at least eight surfaces.

In an embodiment, information about the membrane protein relates to the structure or conformation of the membrane protein or, where a ligand is present, the stoichiometry of a complex in which the membrane protein is bound to the ligand.

In an embodiment, different solubilising agents are present in the electrospray. Different solubilising agents may have different effects on the structural characteristics of a membrane protein, including on membrane proteins in the form of a complex in which the membrane protein is bound to a ligand. A method of the present invention therefore enables *e.g.* rapid and high throughput detergent exchange experiments to be conducted on a membrane protein.

In another embodiment, different ligands are present in the electrospray. For instance, different ligands may be present in the electrospray, and the information about the effect of different ligands on a membrane protein, preferably a membrane protein in the form of a complex in which the membrane protein is bound to a therapeutic agent, may be derived.

A method of the present invention may also enable the dissociation constant of a complex which comprises a membrane protein bound to a ligand to be determined quicker than with conventional mass spectrometry methods. A method for determining the dissociation constant of a complex which comprises a membrane protein bound to a ligand using a desorption electrospray ionisation source coupled to a mass spectrometer may comprise: desorbing membrane proteins from a plurality of surfaces, on each of which a sample comprising a membrane protein, ligand and detergent is deposited, each sample containing a different ratio of membrane protein to ligand, by applying electrospray to the samples; ionising the membrane proteins; detecting the membrane proteins using the mass spectrometer; and calculating the dissociation constant of the complex based on the proportion of membrane proteins that are detected in the form of a complex in which the membrane protein is bound to the ligand from each of the samples.

The plurality of surfaces may together form one larger surface, *i.e.* the plurality of surfaces may be areas of a larger surface. It will be appreciated that the same membrane protein is present in each of the samples. In an embodiment, the plurality of surfaces includes at least two, *e.g.* at least four, *e.g.* at least eight surfaces.

The following non-limiting Examples illustrate the present invention. In the Examples, an electrospray ionisation source was coupled with a ThermoScientific^{™} Q-Exactive orbitrap mass spectrometer in an arrangement similar to that shown in Figure 1 to form a desorption electrospray ionisation apparatus. The electrospray ionisation source comprised a nebulising gas source (N₂). The nebulising gas source - consisting of a stainless steel tee union with a fused silica capillary having a 100 µm internal diameter and a 375 µm outer diameter fitted inside PEEK tubing having a 500 µm internal diameter and a 1600 µm outer diameter - was mounted to an x-y-z translation and rotating stage provided by Newport. The electrospray solution was introduced from a Hamilton syringe using a syringe pump. Voltage was applied to the electrospray solution before it was aerosolised by the nebulising gas.

Solid samples of membrane protein were prepared by electrospraying 25 µL of a 15 µM solution of membrane protein on a UV-cleaned glass cover slip (Menzel-Gläser) at a flow rate of 1 µM/min with a nebulising gas pressure of 250 kPa, and drying the samples using the nebulising gas. Immediately before electrospraying onto the glass slip, the solutions of membrane protein were prepared by buffer exchange using biospin-6 (Bio-Rad) columns equilibrated with the appropriate buffer.

The following membrane proteins were used in the Examples: Outer Membrane Protein F (OmpF), deposited on the glass cover slip in the presence of octyl glucoside detergent; ferric hydroxamate receptor (FvpA) in complex with pyoverdine (pvd), also deposited on the glass cover slip in the presence of octyl glucoside detergent; and semiSWEET, recombinantly expressed (see Lee et al, Nat. Commun. 2015, 6, 6112) and deposited on the glass cover slip in the presence of n-dodecyl β-D-maltoside.

Samples were analysed using the following desorption electrospray ionisation apparatus conditions:

| | |
|---|---|
| Spray voltage | 4 to 5 kV |
| Solvent flow rate | 2 to 3 µL/min |
| Nebulizing gas pressure | 800 to 1000 kPa |
| Spray angle | 55 to 65 ° |
| Spray to sample distance | 2 to 4 mm |
| Sample to capillary distance | 1 to 2 mm |
| Extended trapping energy | 100 to 200 eV |
| Source fragmentation energy | 0 to 100 eV |
| High vacuum pressure | 1 × 10⁻⁴ to 2 × 10⁻⁴ mPa |

### Example 1

OmpF was desorbed from a glass slip and ionised using 100 mM ammonium acetate electrospray solutions containing: (A) 1 % by weight octyl glucoside, a detergent; (B) 50 µM kanamycin sulfate (Sigma Aldrich), an antibiotic; (C) 2.6 µM 1-palmitoyl-2-oleoyl-*sn-*glycero-3-phospho-(1'-*rac*-glycerol) sodium salt (Avanti Polar Lipids), a lipid; and D) 50 µM OBS, a synthetic peptide mimicking a colicin E9 binding epitope.

Figure 2 shows desorption electrospray ionisation mass spectra of OmpF that were obtained using the different electrospray ionisation solutions. It can be seen from the spectra that methods of the present invention enabled the membrane protein to be detected as an intact trimer (see Figure 2A) and in the form of a complex in which the membrane protein is bound to various ligands (see Figures 2B to D).

### Example 2

FvpA in the form of a complex with pvd was desorbed from a glass slip and ionised using 100 mM ammonium acetate electrospray solutions containing 1 % by weight octyl glucoside. For comparison, a micellar solution of FvpA in the form of a complex with pvd was vaporised and ionised using a nanoelectrospray ionisation apparatus.

Figure 3 shows mass spectra of FvpA desorbed from the glass slip using an electrospray solution (see Figure 3A), and vaporised from solution using nanoelectrospray (see Figure 3B). The insets show zero-charge deconvoluted spectra and the calculated percentage of FvpA that was detected in the form of an FvpA-pvd complex. It can be seen that no significant disruption of the interactions between the membrane protein and the ligand occurred throughout the desorption, ionisation and detection process.

### Example 3

SemiSWEET was desorbed from a glass slip and ionised using 200 mM ammonium acetate solutions containing different detergents: A) 0.02 % by weight *n*-dodecyl β-D-maltoside obtained; B) 0.05 % by weight lauryl dimethylamine-N-oxide; and C) 0.5 % by weight tetraethylene glycol monooctyl ether. For comparison, a micellar solution of semiSWEET was vaporised and ionised using a nanoelectrospray ionisation apparatus.

Figure 4 shows mass spectra of semiSWEET that were obtained using the different electrospray ionisation methods (see Figures 4A to C). A mass spectrum of the vaporised micellar solution of semiSWEET is also shown (see the inset of Figure 4A). An extracted ion chromatogram of a 3303 m/z dimer peak from semiSWEET desorbed with the tetraethylene glycol monooctyl ether solution is also shown (see Figure 4D). The results demonstrate that detergent exchange experiments may be performed using desorption electrospray ionisation techniques to observe changes in the stoichiometry of the membrane protein.

### Example 4

In a further experiment, the present methods were used to screen membrane protein-drug interactions. Briefly, the G-protein coupled receptor P2Y₁ was first desorbed in its apo form using a mixed micelle of n-dodecyl β-D-maltoside (DDM), phoscholine and cholesterol (see Figure 5a). This solution was then supplemented with a mixture of 6 ligands (shown below in Table 1) at 20 µM that are designed to target related GPCRs, and only binding of MRS2500 was identified (see Figure 5b). The antagonists listed in Table 1 were selected to represent a possible candidate pool in which only one element (MRS2500; TOCRIS Bio-Techne) is an active antagonist, and were prepared as 1 mM stocks in dd-H₂O. To confirm the specificity towards this ligand, the experiment was repeated with a similar mixture that lacked MRS2500, and the holo peak identified in the previous experiment was absent (see Figure 5c).

**Table 1 - Antagonists used in P2Y₁ drug screening experiments**

| **Antagonist** | **Structure** | **Mass** | **Target** |
|---|---|---|---|
| MRS2500 | 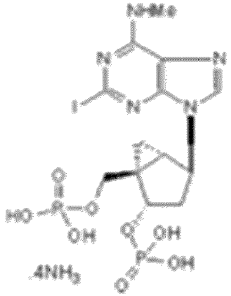 | 561.16 | P2Y₁ |
| MRS2211 | 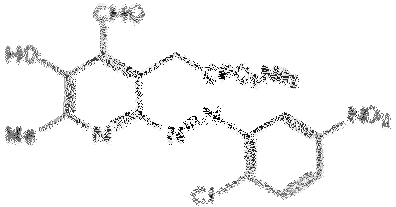 | 428.68 | P2Y1₃ |
| PSB 0739 | 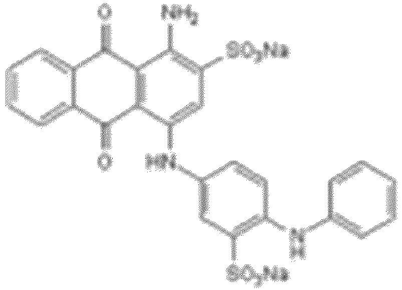 | 563.56 | P2Y1₂ |
| PSB 1115 | 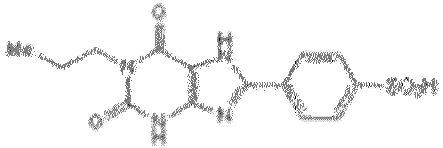 | 350.35 | A2B |
| Ticlopidine Hydrochloride | 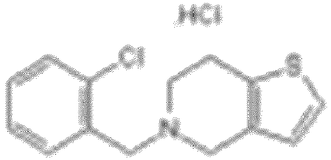 | 263.79 | P2Y1₂ |

### Example 5

In a further experiment, the membrane protein OmpF was desorbed directly from a membrane environment. OmpF was overexpressed in BE3000 cells, in which a very high concentration of protein in the outer membrane can be achieved. The membranes were separated from cell lysate by ultracentrifugation and desalted by resuspension in 200 mM ammonium acetate and subsequent pelleting, which was repeated 5 times. The washed membranes were then extruded through 200 nm to give small unilamellar vesicles. These samples were deposited on slides and desorbed with a 200 mM ammonium acetate with 1% octyl β-D-glucopyranoside (OG) detergent, in order to promote partial disruption of the vesicles during desorption. Using elevated instrument energies, including a source and HCD energies of 150 V and 200 V respectively, the intact OmpF trimer was ejected and detected from the vesicles (see Figure 6).

## Claims

1. A method for detecting a membrane protein using a desorption electrospray ionisation source coupled to a mass spectrometer, wherein the method comprises:
desorbing a membrane protein from a surface on which a sample comprising the membrane protein and a solubilising agent is deposited by applying electrospray to the sample;
ionising the membrane protein; and
detecting the membrane protein using the mass spectrometer.

2. A method according to claim 1, wherein the sample comprises a detergent or lipid solubilising agent, and preferably a membrane protein embedded in a lipid bilayer.

3. A method according to claim 2, wherein the sample comprises a detergent solubilising agent, preferably a non-ionic detergent solubilising agent.

4. A method according to any preceding claim, wherein the membrane protein is an integral membrane protein.

5. A method according to any preceding claim, wherein the sample and/or the electrospray comprises a ligand, and the method comprises ionising and detecting the membrane protein in the form of a complex in which the membrane protein is bound to the ligand, for instance the electrospray comprises a plurality of different ligands, and the method comprises ionising and detecting the membrane protein in the form of a complex in which the membrane protein is bound to at least one of the ligands.

6. A method according to claim 5, wherein the ligand is a therapeutic agent, a nucleotide or a lipid.

7. A method according to any preceding claim, wherein the electrospray comprises a solubilising agent, preferably a detergent or lipid solubilising agent.

8. A method according to any preceding claim, wherein the sample is a solid sample.

9. A method according to claim 8, wherein the solid sample is obtainable by a method in which the solvent is removed from a solution comprising the solubilising agent and the membrane protein, and the solution preferably comprises a detergent micelle in which the membrane protein is contained.

10. A method according to any preceding claim, wherein the membrane protein is detected in a form in which it is substantially intact.

11. A method according to any preceding claim, wherein the structure or conformation of the membrane protein is characterised.

12. A method for deriving information about a membrane protein using a desorption electrospray ionisation source coupled to a mass spectrometer, wherein the method comprises:
desorbing a membrane protein from a plurality of surfaces, on each of which a sample which comprises the membrane protein and a solubilising agent is deposited, by applying electrospray to the samples, wherein a different ligand and/or solubilising agent is present in the electrospray that is applied to each of the samples;
ionising the membrane proteins;
detecting the membrane proteins using the mass spectrometer; and
deriving information about the membrane protein based on the form in which the membrane protein is detected from each of the plurality of samples.

13. A method according to claim 12, wherein the information about the membrane protein relates to the structure or conformation of the membrane protein or, where a ligand is present, the stoichiometry of a complex in which the membrane protein is bound to the ligand.

14. A method for determining the dissociation constant of a complex which comprises a membrane protein bound to a ligand using a desorption electrospray ionisation source coupled to a mass spectrometer, wherein the method comprises:
desorbing membrane proteins from a plurality of surfaces, on each of which a sample which comprises a membrane protein, ligand and detergent is deposited, each sample containing a different ratio of membrane protein to ligand, by applying electrospray to the samples;
ionising the membrane proteins;
detecting the membrane proteins using the mass spectrometer; and
calculating the dissociation constant of the complex based on the proportion of membrane proteins that are detected in the form of a complex in which the membrane protein is bound to the ligand from each of the samples.

## Patentansprüche

1. Ein Verfahren zum Nachweis eines Membranproteins unter Verwendung einer Desorptions-Elektrospray-Ionisationsquelle, die mit einem Massenspektrometer gekoppelt ist, wobei das Verfahren Folgendes beinhaltet:
Desorbieren eines Membranproteins von einer Oberfläche, auf der eine das Membranprotein und einen Lösungsvermittler beinhaltende Probe abgeschieden wird, indem Elektrospray auf die Probe aufgebracht wird;
Ionisieren des Membranproteins; und
Nachweisen des Membranproteins unter Verwendung des Massenspektrometers.

2. Verfahren nach Anspruch 1, wobei die Probe einen Detergens- oder Lipid-Lösungsvermittler und bevorzugt ein in eine Lipiddoppelschicht eingebettetes Membranprotein beinhaltet.

3. Verfahren nach Anspruch 2, wobei die Probe einen Detergens-Lösungsvermittler, bevorzugt einen nichtionischen Detergens-Lösungsvermittler, beinhaltet.

4. Verfahren nach einem der vorherigen Ansprüche, wobei das Membranprotein ein integrales Membranprotein ist.

5. Verfahren nach einem der vorherigen Ansprüche, wobei die Probe und/oder das Elektrospray einen Liganden beinhaltet und das Verfahren das Ionisieren und Nachweisen des Membranproteins in Form eines Komplexes beinhaltet, in dem das Membranprotein an den Liganden gebunden ist, wobei zum Beispiel das Elektrospray eine Vielzahl verschiedener Liganden beinhaltet und das Verfahren das Ionisieren und Nachweisen des Membranproteins in Form eines Komplexes, in dem das Membranprotein an mindestens einen der Liganden gebunden ist, beinhaltet.

6. Verfahren nach Anspruch 5, wobei der Ligand ein Therapeutikum, ein Nukleotid oder ein Lipid ist.

7. Verfahren nach einem der vorherigen Ansprüche, wobei das Elektrospray einen Lösungsvermittler, bevorzugt einen Detergens- oder Lipid-Lösungsvermittler, beinhaltet.

8. Verfahren nach einem der vorherigen Ansprüche, wobei die Probe eine feste Probe ist.

9. Verfahren nach Anspruch 8, wobei die feste Probe durch ein Verfahren erhalten werden kann, in dem das Lösemittel von einer den Lösungsvermittler und das Membranprotein beinhaltenden Lösung entfernt wird, und die Lösung bevorzugt eine Detergens-Micelle beinhaltet, in der das Membranprotein enthalten ist.

10. Verfahren nach einem der vorherigen Ansprüche, wobei das Membranprotein in einer Form nachgewiesen wird, in der es im Wesentlichen intakt ist.

11. Verfahren nach einem der vorherigen Ansprüche, wobei die Struktur oder Konformation des Membranproteins charakterisiert ist.

12. Verfahren zum Ableiten von Informationen über ein Membranprotein unter Verwendung einer Desorptions-Elektrospray-Ionisationsquelle, die mit einem Massenspektrometer gekoppelt ist, wobei das Verfahren Folgendes beinhaltet:
Desorbieren eines Membranproteins von einer Vielzahl von Oberflächen, auf jede von denen eine Probe, die ein Membranprotein und ein Lösungsvermittler beinhaltet, abgeschieden wird, indem Elektrospray auf die Proben aufgebracht wird, wobei ein verschiedener Ligand und/oder Lösungsvermittler in dem Elektrospray vorhanden ist, das auf jede von den Proben aufgebracht wird;
Ionisieren der Membranproteine;
Nachweisen der Membranproteine unter Verwendung des Massenspektrometers; und
Ableiten von Informationen über das Membranprotein auf der Grundlage der Form, in der das Membranprotein aus jeder von der Vielzahl von Proben nachgewiesen wird.

13. Verfahren nach Anspruch 12, wobei die Informationen über das Membranprotein auf die Struktur oder Konformation des Membranproteins, oder, wo ein Ligand vorhanden ist, auf die Stöchiometrie eines Komplexes bezogen ist, in dem das Membranprotein an den Liganden gebunden ist.

14. Ein Verfahren zum Bestimmen der Dissoziationskonstante eines Komplexes, der ein Membranprotein beinhaltet, das an einen Liganden gebunden ist, unter Verwendung einer Desorptions-Elektrospray-Ionisationsquelle, die mit einem Massenspektrometer gekoppelt ist, wobei das Verfahren Folgendes beinhaltet:
Desorbieren von Membranproteinen von einer Vielzahl von Oberflächen, auf jede von denen eine Probe, die ein Membranprotein, einen Liganden und ein Detergens beinhaltet, abgeschieden wird, wobei jede Probe ein verschiedenes Verhältnis von Membranprotein zu Ligand enthält, indem Elektrospray auf die Proben aufgebracht wird;
Ionisieren der Membranproteine;
Nachweisen der Membranproteine unter Verwendung des Massenspektrometers; und
Berechnen der Dissoziationskonstante des Komplexes auf der Grundlage des Anteils an Membranproteinen, die in Form eines Komplexes, in dem das Membranprotein an den Liganden gebunden ist, von jeder der Proben nachgewiesen werden.

## Revendications

1. Procédé de détection d'une protéine membranaire en utilisant une source de désorption-ionisation par électronébulisation couplée à un spectromètre de masse, le procédé comprenant :
la désorption d'une protéine membranaire à partir d'une surface sur laquelle un échantillon comprenant la protéine membranaire et un agent solubilisant est déposé par application d'une électronébulisation sur l'échantillon ;
l'ionisation de la protéine membranaire ; et
la détection de la protéine membranaire à l'aide du spectromètre de masse.

2. Procédé selon la revendication 1, dans lequel l'échantillon comprend un détergent ou un agent de solubilisation des lipides, et de préférence une protéine membranaire incorporée dans une bicouche lipidique.

3. Procédé selon la revendication 2, dans lequel l'échantillon comprend un agent solubilisant détergent, de préférence un agent solubilisant détergent non ionique.

4. Procédé selon l'une quelconque des revendications précédentes, dans lequel la protéine membranaire est une protéine membranaire intégrale.

5. Procédé selon l'une quelconque des revendications précédentes, dans lequel l'échantillon et/ou l'électronébulisation comprennent un ligand, et le procédé comprend l'ionisation et la détection de la protéine membranaire sous la forme d'un complexe dans lequel la protéine membranaire est liée au ligand, par exemple l'électronébulisation comprend une pluralité de différents ligands, et le procédé comprend l'ionisation et la détection de la protéine membranaire sous la forme d'un complexe dans lequel la protéine membranaire est liée à au moins un des ligands.

6. Procédé selon la revendication 5, dans lequel le ligand est un agent thérapeutique, un nucléotide ou un lipide.

7. Procédé selon l'une quelconque des revendications précédentes, dans lequel l'électronébulisation comprend un agent solubilisant, de préférence un agent détergent ou un agent de solubilisation des lipides.

8. Procédé selon l'une quelconque des revendications précédentes, dans lequel l'échantillon est un échantillon solide.

9. Procédé selon la revendication 8, dans lequel l'échantillon solide peut être obtenu par un procédé dans lequel le solvant est éliminé d'une solution comprenant l'agent solubilisant et la protéine membranaire, et la solution comprend de préférence une micelle détergente dans laquelle est contenue la protéine membranaire.

10. Procédé selon l'une quelconque des revendications précédentes, dans lequel la protéine membranaire est détectée sous une forme dans laquelle elle est sensiblement intacte.

11. Procédé selon l'une quelconque des revendications précédentes, dans lequel la structure ou la conformation de la protéine membranaire est **caractérisée**.

12. Procédé d'obtention d'informations sur une protéine membranaire en utilisant une source de désorption-ionisation par électronébulisation couplée à un spectromètre de masse, le procédé comprenant :
la désorption d'une protéine membranaire à partir d'une pluralité de surfaces, sur chacune desquelles un échantillon qui comprend la protéine membranaire et un agent solubilisant est déposé, par application d'une électronébulisation sur les échantillons, un différent ligand et/ou agent solubilisant étant présent dans l'électronébulisation qui est appliquée sur chacun des échantillons ;
l'ionisation des protéines membranaires ;
la détection des protéines membranaires à l'aide du spectromètre de masse ; et
l'obtention d'informations sur la protéine membranaire en fonction de la forme sous laquelle la protéine membranaire est détectée à partir de chacun des échantillons de la pluralité.

13. Procédé selon la revendication 12, dans lequel les informations sur la protéine membranaire se rapportent à la structure ou à la conformation de la protéine membranaire ou, lorsqu'un ligand est présent, à la stœchiométrie d'un complexe dans lequel la protéine membranaire est liée au ligand.

14. Procédé de détermination de la constante de dissociation d'un complexe qui comprend une protéine membranaire liée à un ligand en utilisant une source de désorption-ionisation par électronébulisation couplée à un spectromètre de masse, le procédé comprenant :
la désorption des protéines membranaires à partir d'une pluralité de surfaces, sur chacune desquelles est déposé un échantillon qui comprend une protéine membranaire, un ligand et un détergent, chaque échantillon contenant un différent rapport de la protéine membranaire au ligand, par application d'une électronébulisation sur les échantillons ;
l'ionisation des protéines membranaires ;
la détection des protéines membranaires à l'aide du spectromètre de masse ; et
le calcul de la constante de dissociation du complexe en fonction de la proportion des protéines membranaires qui sont détectées sous la forme d'un complexe dans lequel la protéine membranaire est liée au ligand à partir de chacun des échantillons.
